# EUROPÄISCHE PATENTSCHRIFT

(11) **EP 3 013 972 B1**
(45) Veröffentlichungstag und Bekanntmachung des Hinweises auf die Patenterteilung: **22.08.2018**
(21) Anmeldenummer: 14744750.2
(22) Anmeldetag: 10.06.2014
(51) Int. Cl.: G01N 33/50, G16H 15/00, G16H 50/70

(54) **VERFAHREN ZUR ERSTELLUNG EINER DATENBANK FÜR DIE VORABSCHÄTZUNG DER WIRKSAMKEIT VON WIRKSTOFFEN IN DER TUMORTHERAPIE**
METHOD FOR CREATING A DATABASE FOR THE PRELIMINARY ESTIMATION OF THE EFFECTIVENESS OF ACTIVE SUBSTANCES IN TUMOR THERAPY
PROCÉDÉ DE CRÉATION D'UNE BASE DE L'ESTIMATION PRÉLIMINAIRE DE L'EFFICACITÉ DES SUBSTANCES ACTIVES DANS LE TRAITEMENT DES TUMEURS

(30) Priorität: 25.06.2013 DE 102013010528
(43) Veröffentlichungstag der Anmeldung: 04.05.2016
(73) Patentinhaber: FLACOD GmbH, 69121 Heidelberg (DE)
(72) Erfinder: GRANZOW, Christof, 69121 Heidelberg (DE)
(74) Vertreter: Rudolph, Ulrike
(86) Internationale Anmeldenummer: PCT/DE2014/000293
(87) Internationale Veröffentlichungsnummer: WO 2014/206387

(56) Entgegenhaltungen:
- WO-A1-98/02038
- WO-A1-2013/044099
- DE-A1- 19 520 729
- DE-A1-102009 047 146
- DE-B3-102012 003 700
- US-A1- 2004 072 722
- US-A1- 2009 104 647
- STAIB P ET AL: "PREDICTION OF INDIVIDUAL RESPONSE TO CHEMOTHERAPY IN PATIENTS WITH ACUTE MYELOID LEUKAEMIA USING THE CHEMOSENSITIVITY INDEX CI", BRITISH JOURNAL OF HAEMATOLOGY, WILEY-BLACKWELL PUBLISHING LTD, GB, Bd. 128, Nr. 6, 1. März 2005 (2005-03-01), Seiten 783-791, XP002433893, ISSN: 0007-1048, DOI: 10.1111/J.1365-2141.2005.05402.X
- ANDREOTTI P E ET AL: "CHEMOSENSITIVITY TESTING OF HUMAN TUMORS USING A MICROPLATE ADENOSINE TRIPHOSPHATE LUMINESCENCE ASSAY: CLINICAL CORRELATION FORCISPLATIN RESISTANCE OF OVARIAN CARCINOMA", CANCER RESEARCH, AMERICAN ASSOCIATION FOR CANCER RESEARCH, US, vol. 55, 15 November 1995 (1995-11-15), pages 5276-5282, XP000887217, ISSN: 0008-5472
- B Desoize ET AL: "Evaluation of a Prediction Model of Cisplatin Dose Based on Total Platinum Plasma Concentration INTRODUCTION", Eur J Cancer, vol. 32A, 1 September 1996 (1996-09-01), pages 1734-1738, XP055465913,

## Beschreibung

Die Erfindung betrifft ein Verfahren zur Erstellung einer Datenbank für die Vorabschätzung (Voreinschätzung, Prognostizierung) der Wirksamkeit eines (zytostatischen) Wirkstoffs oder einer (zytostatischen) Wirkstoff-Kombination bei der Chemotherapie eines Tumors eines betreffenden Patienten sowie Verfahren zur Vorabtestung eines geplanten Wirkstoffs oder einer geplanten Wirkstoff-Kombination ex-vivo und Prognostizierung von dessen Wirksamkeit (therapeutischer Wirkung) auf den Tumor eines bestimmten Patienten unter Verwendung dieser Datenbank.

Malignome (Synonym: Krebstumoren) sind bösartige (maligne) Tumoren, die der normalen Wachstumskontrolle des Organismus entzogen sind, sich ungehindert vermehren, in das umliegende Gewebe eindringen und es zerstören, und die metastasieren können, d.h. sie können in Blut- und Lymphgefäße eindringen und über diese in andere Körperorgane gelangen, wo sie sich wieder ansiedeln und vermehren (d.h. Metastasen bilden).
Demgegenüber zeichnen sich die gutartigen (benignen) Tumoren dadurch aus, dass sie nicht invasiv in das umliegende Gewebe einwachsen, sondern es nur verdrängen, und dass sie keine Metastasen bilden.

Obwohl in der Malignom-Therapie (Synonym: Krebs-Therapie) die Behandlung mit Bestrahlung oder mit der Einnahme von Zytostatika oder mit einer Kombination beider Methoden seit Jahrzehnten angewendet wird, beträgt die Erfolgsquote bei den meisten Karzinomen noch immer nur etwa 20 Prozent.
Insbesondere bei der Chemotherapie besteht das Problem, dass eine Behandlung mit einem unpassenden (falschen) oder zu niedrig dosierten Chemotherapeutikum nicht nur keine malignomhemmende Wirkung hat, sondern darüber hinaus zu einer erhöhten Therapieresistenz des Malignoms führen kann. Im Fall von zwei oder mehreren Chemotherapiezyklen mit demselben Chemotherapeutikum, was im Stand der Technik gängige Maßnahme ist, sind die Erfolgsraten nach der zweiten und weiteren Behandlung folglich in der Regel noch viel niedriger, als nach der ersten Behandlung.
Gegenwärtig sind die allermeisten Behandlungspläne in der Chemo- und Strahlentherapie mehr oder weniger empirisch definiert, was schon deshalb problematisch ist, weil jedes Malignom eines Individuums auf ein Chemotherapeutikum und/oder eine Bestrahlung individuell reagiert.
Für eine effektive und auf das spezifische Malignom eines Individuums zugeschnittene Therapie wurden aber auch schon Testverfahren vorgeschlagen, mit denen vor Verabreichung einer geplanten Chemo- und/oder Strahlentherapie die Sensitivität der Malignomzellen auf das geplante Therapeutikum, insbesondere das Chemotherapeutikum, geprüft werden kann, z.B. in der US 20010051353 A1 und in der WO 2009124997 A1. Die Durchführung eines solchen Testverfahrens erfolgt in vitro, vorzugsweise ex vivo, d.h. an einer frisch aus dem Organismus isolierten Malignomgewebeprobe, wie z.B. gemäß WO 2009124997 A1 oder DE 102012003700 B3 (während die US 20010051353 A1 eine über mehrere Wochen an- bzw. hochgezüchtete Zell-Monoschicht-Kulturen vorschlägt und verwendet).
Durch Inkubation mit verschiedenen Konzentrationen des Therapeutikums kann dessen IC50-Wert bestimmt werden. Der IC50-Wert eines Chemotherapeutikum steht für diejenige Konzentration dieses Mittels, bei der die Proliferation der damit behandelten und untersuchten Zellen im Vergleich zu unbehandelten Kontrollzellen auf 50% reduziert ist. Er ist ein wichtiges Kriterium für die Beschreibung der Malignomreaktion auf ein Chemotherapeutikum.
Anhand der Testergebnisse lässt sich abschätzen, wie stark empfindlich die Malignomzellen für das Therapeutikum sind. US2009/0104647 offenbart Methoden zur Individualisierung der Chemotherapie bei Krebspatienten. Da in den letzten Jahren nachgewiesen wurde, dass nicht nur die malignen Zellen eines Malignoms zur Resistenzentwicklung befähigt sind, sondern auch oder sogar nur die Zellen des Malignomstromas, z.B. bei primären und metastatischen Lungentumoren (Granzow et al., 2004) und in Kopf-Hals-Karzinomen (Dollner et al., 2004), sollten die Tumorgewebeproben vor der Inkubation mit dem zu testenden Wirkstoff einem Verfahren zur Identifizierung - und gegebenenfalls auch Separierung - und Diffenrenzierung von malignen Zellen einerseits und Stromazellen (insbesondere Endothelzellen und Fibroblasten) andererseits unterworfen werden. So kann anschließend die jeweilige Reaktion der jeweiligen Zellgruppe auf den zu untersuchenden Wirkstoff getestet werden. Die Ergebnisse eines solchen Wirkstoff-Wirksamkeitstests, insbesondere die IC50-Werte der Malignomzellen der Tumorgewebeprobe eines betreffenden Patienten, dienen im Stand der Technik als ein grober Anhaltspunkt dafür, wie der Behandlungsplan für diesen Patienten bezüglich Dosis und Verabreichungszyklen gewählt werden sollte, um den gewünschten therapeutischen Effekt zu erreichen.
Da eine Chemotherapie zwar die Chancen birgt, den Tumor signifikant zu hemmen, sie aber auch mit einer deutlichen Toxizität für andere Zellen des damit behandelten Organismus (d.h. des Patienten) einhergeht, sollte sie so genau wie möglich auf den betreffenden Patienten und dessen Tumor abgestimmt sein. Sowohl die Art als auch die Dosierung des eingesetzten Wirkstoffs oder der eingesetzten Wirkstoffe/Wirkstoffkombination sollte so gewählt sein, dass einerseits der Tumor mit hoher Wahrscheinlichkeit zur Regression (Rückbildung) oder zumindest zum Wachstumsstillstand gebracht wird, und dass andererseits die übrigen Zellen des Patientenorganismus so wenig wie möglich mit Toxizität belastet werden.
Für eine derart patientenspezifische, individualisierte Therapiekonzipierung fehlen in der Praxis jedoch bisher geeignete Daten- bzw. Informationsquellen, die einen schnellen und gezielten Zugriff in jedem Einzelfall eines konkreten Patienten ermöglichen.
Der vorliegenden Erfindung liegt die Aufgabe zugrunde, diese Nachteile des Stands der Technik zu beheben oder wenigstens zu mindern.

Eine Lösung der genannten Aufgabe besteht in der Bereitstellung eines Verfahrens zur Erstellung einer Datenbank für die Vorabschätzung der Wirksamkeit eines zytostatischen Wirkstoffs, der ein Platinderivat/Platinanalogon ist, in der chemotherapeutischen Behandlung eines Tumors, insbesondere eines Malignoms, eines bestimmten Patienten anhand der ex vivo Ermittlung des IC50 Wertes des Wirkstoffs für die Tumorzellen einer zuvor entnommenen Tumorgewebeprobe desselben Patienten, gekennzeichnet durch folgende Maßnahmen:- aus der Tumorgewebeprobe wird durch enzymatischen Verdau mit Kollagenase bei 36-37°C für 4-5 Stunden, Zentrifugieren und Resuspendieren ein Tumordigest hergestellt, das Zellverbände aus malignen Zellen einerseits und Stromazellen, d. h. Endothelzellen und Fibroblasten, andererseits enthält,-die Zellverbände werden ex vivo mit dem betreffenden Wirkstoff inkubiert durch Zugabe von Aliquots dieses Tumordigests zu vorab doppelt konzentriert angesetzten gleichgroßen Aliquots von Wirkstoff in Kulturmedium;- der IC50-Wert dieses Wirkstoffs bei den malignen Zellen wird ermittelt als diejenige Konzentration des Wirkstoffs, bei der im Vergleich zu Negativ-Kontrolle (0 µM Wirkstoff) eine halbmaximale Inhibition der Koloniebildung beobachtbar ist;- dem ex vivo ermittelten IC50-Wert wird die Dokumentation und/oder Klassifizierung desjenigen Therapieergebnisses zugeordnet, das von demselben Patienten nach Therapie mit demselben Wirkstoff gemäß geltenden evidenz-basierten Therapieregeln stammt und das dokumentiert und klassifiziert worden ist,- die verwendeten Wirkstoffkonzentrationen werden mittels parallel untersuchter etablierter in vitro Zellstämme der gleichen Tumorentität verifiziert. Begriffsdefinitionen im vorliegenden Kontext:
Der Begriff "maligne Zellen" steht für die Zellen eines Malignoms, die autonom proliferieren, zum invasiven-destruktiven Wachstum fähig sind und Metastasen bilden können.
Der Begriff "Stromazellen" steht hier für die Zellen eines Malignoms, die in vivo mit den malignen Zellen ko-existieren, selbst jedoch keine biologische Malignität aufweisen und ex vivo proliferationsfähig sind (z.B. Fibroblasten und zelluläre Komponenten des Blutgefäßsystems, insbesondere Endothelzellen).
Der Begriff "Zellverband" (Plural: "Zellverbände") steht für zellulär weitgehend homogene Aggregationen von Zellen, die ähnlich auch im Gewebe eines Malignoms in situ vorliegen bzw. vorgelegen haben
Der Begriff "Tumordigest" steht für das aus der Tumorgewebeprobe ex vivo mittels Kollagenaseverdau gewonnene Aufschlussprodukt nach Zentrifugieren und Resuspendieren in Kulturmedium.

Der Begriff "Zelllinie" steht für eine Primärkultur (d.h. eine von Zellen, Geweben oder Organen angelegte Kultur) zum Zeitpunkt der ersten Subkultur.
Der Begriff "Zellstamm" steht für Zellen, die durch Selektion oder Klonen aus einer Primärkultur oder einer Zelllinie gewonnen wurden und charakteristische Merkmale aufweisen, die während einer nachfolgenden Kultivierung bzw. Subkultivierung fortbestehen.
Die Abkürzung "g" steht für die mittlere Erdbeschleunigung.
Der Begriff "IC 50-Wert" steht für die mittlere inhibitorische Konzentration (IC₅₀) eines Inhibitors (allgemein: eines Wirkstoffs), d.h. diejenige Konzentration, bei der eine halbmaximale Inhibition beobachtet wird. Nach der FDA ist die IC₅₀ die Konzentration einer Substanz, die notwendig ist, um bei einem Target in vitro eine 50%ige Inhibierung zu erreichen. Im Fall einer Koloniebildung von Zellen einer Tumorgewebeprobe (Zellkolonie) ist der IC50-Wert also diejenige Konzentration eines Wirkstoffs / Inhibitors, bei der im Vergleich zu Negativ-Kontrolle (0 µM Wirkstoff) eine halbmaximale Inhibition der Koloniebildung (des jeweiligen Zelltyps) beobachtet wird.
Die Konzentrationsangaben "M", "mM" und "µM" beziehen sich auf Mol pro Liter, Millimol pro Liter und Mikromol pro Liter.
Die erfindungsgemäße Datenbank ermöglicht ein gegenüber dem Stand der Technik wesentlich verbessertes Verfahren zur Voreinschätzung (Vorabschätzung, Prognostizierung) der Wirksamkeit eines zytostatischen Wirkstoffs, wobei der Wirkstoff oder einer der Wirkstoffe ein Platinderivat/Platinanalogon ist, zur Bekämpfung eines Tumors eines bestimmten Patienten, anhand der ex vivo Ermittlung des IC50-Wertes dieses Wirkstoffs für die Tumorzellen des betreffenden Patienten. Das gilt insbesondere, wenn es sich bei dem Tumor um ein Malignom und weiter insbesondere um ein Karzinom handelt. Bei diesem verbesserten Verfahren werden die gemäß dem zuvor beschriebenen Verfahren gewonnenen Tumorzellverbände einer zuvor entnommenen Tumorgewebeprobe desselben Patienten ex vivo mit dem betreffenden Wirkstoff nach Zugabe von Aliquots des Tumordigests zu vorab doppelt konzentriert angesetzten gleichgroßen Aliquots von Wirkstoff in Kulturmedium inkubiert, und der IC50-Wert dieses Wirkstoffs wird bei den malignen Zellen ermittelt, wobei der IC50 Wert diejenige Konzentration des Wirkstoffs ist, bei der im Vergleich zu Negativ-Kontrolle (0 µM Wirkstoff) eine halbmaximale Inhibition der Koloniebildung beaobachtbar ist. Anschließend wird der ermittelte IC50-Wert mit den in der Datenbank enthaltenen, vorab ermittelten IC50-Referenzwerten des gleichen Wirkstoffs bei Tumorzellen der gleichen Tumorentität verglichen.

Wie bereits ausgeführt, ist in der Datenbank jedem IC50-Referenzwert ein Therapieergebnis zugeordnet, das dadurch erhalten wurde (bzw. erhältlich ist), dass der Patient, mit dessen Tumorzellen der IC50-Referenzwert gewonnen wurde (bzw. wird), mit dem Wirkstoff nach geltenden evidenz-basierten Therapieregeln behandelt wurde (bzw. wird), das resultierende Therapieergebnis dokumentiert und klassifiziert wurde (bzw. wird), beispielsweise in Form einer Klassifizierung in eine der Klassen I = vollständige Remission des Tumors (VR), II = partielle Remission des Tumors (PR), III = Nullwachstum des Tumors = "no change" (NC) und IV = fortschreitendes Wachstum des Tumors = Progress.
Eine Übereinstimmung zwischen dem für die Tumorzellen des aktuellen Patienten ermittelten IC50-Wert und einem der in der Datenbank enthaltenen IC50-Referenzwerte gilt als Indiz dafür, dass das in der Datenbank diesem Referenzwert zugeordnete Therapieergebnis auch im Fall einer Behandlung des aktuellen Patienten eintritt.
Eine Lösung der eingangs genannten Aufgabe besteht somit auch in der Bereitstellung eines solchen Verfahrens zur Voreinschätzung der Wirksamkeit eines zytostatischen Wirkstoffs zur Bekämpfung eines Tumors.

Die Aufarbeitung einer Tumorgewebeprobe für die Inkubation mit dem zu testenden Wirkstoff umfasst vorzugsweise die folgenden Maßnahmen:
Die vorzugsweise frisch gewonnene Tumorgewebeprobe wird zunächst (vorzugsweise binnen maximal 15 Minuten) von nekrotischen Anteilen und anhängendem sonstigem (Nicht-Malignom-) Gewebe gereinigt und zerkleinert (vorzugsweise zerschnitten), vorzugsweise in Stücke von etwa 1 bis 4 mm³ (Kubikmillimeter) Volumen. Das zerkleinerte Gewebe wird in (vorzugsweise Serum-haltigem) Kulturmedium aufgeschwemmt (suspendiert). Die gewonnene Suspension wird einer Behandlung mit Kollagenase (d.h. einem Kollagenaseverdau bzw. einem Kollagenaseaufschluß) bei etwa 36°C bis 37°C für 4-5 Stunden unterworfen. Das dadurch erhaltene Kollagenaseaufschlußprodukt wird zentrifugiert (z.B. 5 Minuten bei 50 x *g* und Raumtemperatur), und das gewonnene Pellet wird vom Überstand abgetrennt.
Dieses Pellet wird in Kulturmedium resuspendiert und das so gewonnene Tumordigest anschließend zusammen mit dem Wirkstoff inkubiert. Dabei ist die zeitliche Reihefolge von Bedeutung, dass die Aliquots des Tumordigests den vorab doppelt konzentriert angesetzten gleich großen Aliquots von Wirkstoff in Kulturmedium zugegeben werden.

In der erfindungsgemässen Ausführung dieses Verfahren zur IC50-Bestimmung werden die üblicherweise rechnerisch (anhand der Herstellerangaben auf der Stocklösung) ermittelten Konzentrationen der Wirkstoff-in-Kulturmedium-Aliquots in einem Verifizierungstest überprüft. Dieser Verifizierungstest zeichnet sich dadurch aus, dass von einigen Verdünnungsstufen der aus der Wirkstoff-Stocklösung und Kulturmedium hergestellten Verdünnungsreihe sowohl Aliquots für die Inkubation und IC50-Bestimmung mit den Zellen des Tumordigests entnommen werden als auch Aliquots für eine parallele IC50-Bestimmung mit Zellen eines etablierten Zellstamms oder einer etablierten Zelllinie jeweils aus einer Primärkultur von Zelle der gleichen Tumorentität wie das Tumordigest. Alle Test-Ansätze, d.h. die mit den Zellen des Tumordigests und die mit den Zellen des Zellstamms oder der Zelllinie, werden parallel (simultan, gleichzeitig) inkubiert und mit den gleichen Analyseverfahren hinsichtlich ihres IC50-Werts analysiert.

Für eine hierin beschriebene separate Behandlung bzw. Testung von malignen Zellen einerseits und/oder Stromazellen (insbesondere Endothelzellen und Fibroblasten) andererseits und jeweils zudem im Zellverband kann das Pellet vor der Inkubation mit dem zu testenden Wirkstoff einem Trennverfahren gemäß DE 102012003700B3 unterworfen werden.
Die Erfindung wird im folgenden anhand von Ausführungsbeispielen und dazugehörigen Tabellen näher erläutert:
Tabelle 1 zeigt: für 18 Bronchialkarzinom-Tumorproben (Spalte 1 "Lfd Nr. 1-18") von 17
   verschiedenen Patienten die pathohistologische Klassifizierung und das
   Stadium des Tumors (Spalte 3), die in der dem Patienten verabreichten
   Chemotherapie verwendeten Cytostatika (Spalte 4) und den am Patienten
   klinisch diagnostizierten Therapieerfolg (Spalte 5).
   SCLC = kleinzelliges Bronchialkarzinom
   NSCLC = nichtkleinzelliges Bronchialkarzinom
   CarboPt = Carboplatin
   CisPt = Cisplatin
   PR = partielle Remission (d.h. das Karzinom ist nachweisbar kleiner geworden)
   NC = "No change" = Null-Wachstum des Karzinoms
   Progress = das Karzinom ist weiter gewachsen, kein Therapieerfolg
   Die Tumoren ID 278 und ID 279 stammten vom gleichen Patienten. Es handelte sich um unabhängig voneinander existierende, nichtkleinzellige Primärtumoren. ID 278 war ein Adenokarzinom, ID 279 ein Plattenepithelkarzinom.
Tabelle 2 zeigt: die Korrelation der prätherapeutisch ermittelten Cisplatin-IC50-Werte der Tumorzellen ex vivo mit den Ergebnissen der platinbasierten Chemotherapie beim Patienten in vivo. Unterscheidbare Konstellationen sind:
   (Patienten-) Gruppe (1):
      niedrige IC50-Werte (unter 5 µM Cisplatin), d.h.: hohe Empfindlichkeit (Sensibilität) der Zellen gegenüber diesem Wirkstoff;
      partielle Remission (PR) des Tumors, d.h. guter Therapieerfolg;
   (Patienten-) Gruppe (2):
      mittlere IC50-Werte (größer oder gleich 5 bis kleiner 8 µM Cisplatin) d.h.: mäßige Empfindlichkeit (Sensibilität) der Zellen gegenüber diesem Wirkstoff;
      partielle Remission (PR) oder Null-Wachstum (NC = "no change") des Tumors, d.h. Therapieerfolg;
   (Patienten-) Gruppe (3):
      - hohe IC50-Werte (8 bis 25,6 µM Cisplatin), d.h.: geringe Empfindlichkeit der Zellen gegenüber diesem Wirkstoff;
      dennoch partielle Remission (PR) oder Null-Wachstum (NC) des Tumors, d.h. Therapieerfolg;
   (Patienten-) Gruppe (4):
      enthält Sonderfälle
Tabelle 3 zeigt: die graphische Darstellung der Datenbank "Therapieerfolgs-Prognose beim Bronchialkarzinom mit dem Zytostatikum Cisplatin"

### Beispiel 1: Erstellung einer Datenbank für die Vorabschätzung der Wirksamkeit des zytostatischen Wirkstoffs Cisplatin in der chemotherapeutischen Behandlung des Bronchialkarzinoms

### (I) Bestimmung der IC50 für Cisplatin in Tumorzellen von Bronchialkarzinom-Gewebeproben, gewonnen von Patienten mit unbehandeltem Bronchialkarzinom

Teile des frisch (dem Patienten in Vollnarkose) entnommenen (hier biopsierten) Bronchialkarzinomgewebes wurden ins Labor gebracht und unverzüglich für die IC50-Bestimmung aufbereitet.
Der Transport ins Labor sollte idealerweise in gekühltem Kulturmedium erfolgen. Hierfür kommt insbesondere HEPES und NaHCO3 gepuffertes, vorzugsweise flavinfreies RPMI 1640 Kulturmedium mit einem Gehalt von 10 % fötalem Rinderserum, 100 IU/ml G-Penicillin, 100 µg/ml Streptomycin, 20 µg/ml Amikacin (als Antibiotika) und 8 µg/ml Voriconazol (als Antimykotikum) in Betracht.

Im Labor wurden diese Tumorgewebeproben gereinigt, gewogen und zerkleinert und in Kulturmedium suspendiert.

Hierfür kommt insbesondere das Verfahren gemäß DE 102012003700 B3 in Betracht, bei dem ca. 5,5 mg Tumorgewebeprobe pro 1 ml Kulturmedium in einer Petrischale unter Standardbedingungen in einem Begasungsbrutschrank (36-37°C, 3,5% CO2 in wasserdampfgesättigter Luft) für 4-5 Stunden einem enzymatischen Verdau mit Kollagenase unterworfen werden, vorzugsweise unter Einsatz von Clostridium histolytikum Kollagenase (Sigma, 230 CDU/ml).
Im Anschluß an den Kollagenaseverdau wird das Aufschlussprodukt bei 50 x ***g*** zentrifugiert, der Überstand verworfen und das Pellet in 1 ml frischem Kulturmedium resuspendiert. Mit dieser Resuspension - im folgenden "Tumordigest" genannt - wird der Chemosensitivitäts-Test (IC50-Bestimmung) durchgeführt

Für den Chemosensitivitäts-Test (IC50-Bestimmung) mit Cisplatin wurden zunächst Cisplatin-Lösungen wie folgt hergestellt: Aus einer 1mM Stocklösung von Cisplatin in Wasser wurden in Kulturmedium Testlösungen mit folgenden Cisplatin-Endkonzentration hergestellt: 0 µM (als Negativ-Kontrolle); 0,1 µM; 0,2 µM; 0,4 µM; 0,8 µM; 1,6 µM; 3,2 µM; 6,4 µM; 12,8 µM und 25,6 µM. Diese rechnerisch anhand der Herstellerangaben auf der Stocklösung ermittelten Cisplatinkonzentrationen wurden in einem Verifizierungstest mit IC50-Bestimmung überprüft.

Für die IC50-Bestimmung in den Tumordigests (Zellsuspensionen aus den Tumorgewebeproben der Patienten) wurde pro Tumordigest in einer 96-Loch-Kulturplatten, die mit extrazellulärer Matrix (ECM) beschichtete waren, ein Doppelansatz (zwei Kavitäten) für jede Cisplatin-Endkonzentration vorbereitet, in dem in jede der beiden Kavitäten jeweils 150µl Cisplatin-Testlösung derselben Cisplatin-Endkonzentration vorgelegt wurden.
Die derart vorbereiteten Kulturplatten wurden unter Standardbedingungen (d.h.: Begasungsbrutschrank; 36-37°C; 3,5% CO₂ in wasserdampfgesättigter Luft) für ca 1 Stunde vorinkubiert.
Anschließend wurden diese vorinkubierten Kavitäten mit jeweils 150 µl Tumordigest versetzt, für 72 Stunden unter Standardbedingungen (.s.o.) inkubiert und anschließend mikroskopisch analysiert.

Für den Verifizierungstest der verwendeten Cisplatin-Konzentrationen wurden anstelle der Patienten-Tumordigests eine Zellsuspension des Lungen-Adenokarzinom-Zellstamms A240286S (Heuser et al., 2005) mit einer Zelldichte von 3x10⁴/ml eingesetzt. In Duplikaten pro angegebene Konzentration wurden jeweils 150 µl der auch für die Tumordigests benutzten Ausgangslösungen mit 0 µM, 0,4 µM, 0,8 µM, 1,6 µM, 3,2 µM und 6,4 µM Cisplatin pro Kavität einer für Zellkultivierung geeigneten 96-Loch-Kulturplatte vorgelegt. Die fertigen Konzentrationsgradienten wurden unter Standardbedingungen (s.o.) für ca. 30 Minuten vorinkubiert. Anschließend wurden pro Loch (Kavität) jeweils 150 µl einer Suspension frisch mit Accutase (bevorzugt Promocell) vereinzelter A240286S-Zellen (Zelldichte: 3 x 10⁴/mL) zu den vorgelegten Cisplatin-Lösungen hinzugefügt. Die Kulturplatte wurde danach parallel zu der mit den Tumordigests bestückten Kulturplatte für 72 Stunden unter Standardbedingungen inkubiert.

Alle Arbeiten mit serumhaltigem Kulturmedium und Cisplatin wurden bei einer Arbeitsplatzbeleuchtung ausschließlich durch Licht von Wellenlängen über 520 nm durchgeführt.

Nach Inkubationsende wurde bei allen Tumordigest-Ansätzen zunächst das Kulturmedium entfernt und die vorhandenen Zellkolonien mit phosphatgepufferter physiologischer Kochsalzlösung (PBS) gespült und abtropfen gelassen. Anschließend wurden die Zellen mit Methanol bei minus 25°C für 5-10 Minuten fixiert und danach einer Giemsa-Färbung nach Gurr unterworfen. Mikroskopisch wurde die durchschnittliche Anzahl der pro Kavität vorhandenen Kolonien epithelialer Tumorzellen für jede Cisplatin-Konzentration einschließlich 0 µM Cisplatin (als Negativ-Kontrolle) ermittelt.

Bei den Ansätzen des Verifizierungstest (mit den Zellen des Lungen-Adenokarzinom-Zellstamms A240286S) wurde nach Inkubationsende das Kulturmedium abgegossen. Die vorliegenden Zellrasen wurden mit PBS gespült, abtropfen gelassen und anschließend mit Accutase vom Kulturgefäß abgelöst. Die Zellen wurden mittels Passieren durch eine stumpfe Kanüle Nr. 20 vereinzelt. Mit Hilfe einer Zell-Zähl-und-Analysier-Apparatur (hier des Casy I Cell Analyzers der Firma Roche, Basel) wurde die Zelldichte jedes Testansatzes ermittelt und um das Inoculum vermindert.

Für die Cisplatin-IC50-Wert-Bestimmung wurden sowohl die mikroskopisch (für die Tumordigestzellen) ermittelten Werte als auch die mittels Zell-Zähl-und-Analysier-Apparatur (für die Zellen des Verifizierungstests) ermittelten Werte logarithmiert und graphisch gegen den Prozentsatz der Negativ-Kontroll-Werte (0 µM Cisplatin) dargestellt. Anschließend wurde mit log-linearen Verfahren (hier im Beispiel mit Verfahren der log-linearen Regression unter Anwendung der Software SAS JMP Version 9.0) in denjenigen Abschnitten der Plots, in denen die relative Häufigkeit von Kolonien mit zunehmender Konzentration von Cisplatin linear abnahm, die IC50-Werte ermittelt.

Die so erhaltenen Cisplatin-IC50-Werte für die Tumorzellen (aus den Tumorgewebeproben) der jeweiligen Patienten sind in Tabelle 2, jeweils Spalte 2 der Patientengruppen 1-4 dargestellt.
Die IC50-Werte der Gruppen 1, 2 und 3 wurden gruppenweise einander gegenüber gestellt und mittels ungepaarten, zweiseitigen t-Tests verglichen. Die resultierenden p-Werte sind in Tabelle 2 angegeben. Gruppe 1 und Gruppe 2 sowie Gruppe 2 und Gruppe 3 zeigen signifikante Unterschiede zu einem für multiples Testen adjustierten zweiseitigen Signifikanzniveau von 0.025 (entspricht Bonferroni- adjustierten Niveaus von 0.05/2). Das bedeutet: Die Unterschiede zwischen Gruppe 1 und Gruppe 2 sowie zwischen Gruppe 2 und Gruppe 3 sind signifikant, wobei letzteres impliziert, dass auch Gruppe 1 von Gruppe 3 signifikant verschieden ist. Die Irrtumswahrscheinlichkeit liegt jeweils unter ein Prozent.

Die erhaltenen Cisplatin-IC50-Werte für die A240286S-Zellen des Verifizierungstests betrugen bei 15 auswertbaren Testansätzen durchschnittliche 1,2 µM ± 0,28 µM.

### (II) Pathohistologische Klassifizierung, Tumorstadium, Chemotherapie und therapeutisches Behandlungsergebnis

Parallel zu aber unabhängig von der Ermittlung der IC50-Werte mit Cisplatin für die Tumorzellen ihrer Tumorgewebeproben im Labor wurden die betreffenden Patienten einer platinbasierten Chemotherapie nach evidenzbasierten Protokollen unterworfen. Gemäß dieser Protokolle wird - und wurde folglich auch hier im Beispiel - Cisplatin oder Carboplatin immer mit einem oder mehreren Nicht-Platin-Zytostatika, hier im Beispiel mit einem oder mehreren der Wirkstoffe Docetaxel, Etoposid, Gemzitabin, Pemetrexed, LY2603618 oder Vinorelbin kombiniert.

Cisplatin und Carboplatin gehören zur Zytostatika-Gruppe der Platinderivate/Platinanaloga, die die Zellvermehrung stören, indem sie Brüche oder Vernetzungen der DNA-Stränge induzieren.
Vinorelbin und Docetaxel hemmen den Zellzyklus, indem sie in den Auf- bzw. Abbau der Kernspindel während der Mitose eingreifen.
Etoposid stört die DNA-Replikation durch Hemmung der Topoisomerase II.
Gemzitabin hemmt nach Aktivierung als Nukleosid-Antimetabolit die DNA-Synthese. Pemetrexed hemmt mehrere folatabhängige Schlüsselenzyme der ne novo Biosynthese von Thymidin- und Purinnukleotiden.
Die Studiensubstanz LY2603618 ist ein ChK 1 Inhibitor, der den DNA-Reparaturmechanismus stört.

Das Behandlungsergebnis, nämlich die Veränderung bzw. der Zustand des Tumors nach zwei bis drei Behandlungszyklen im Vergleich zum Zustand vor Behandlungsbeginn, wurde computertomographisch analysiert und anhand der bekannten und im Stand der Technik üblichen "Standard"-Bewertungsskala als Klasse I = vollständige Remission (VR), Klasse II = partielle Remission (PR), Klasse III = Nullwachstum ("no change", NC) und Klasse IV = Progress (fortschreitendes Tumorwachstum) klassifiziert.

Die erzielten Behandlungsergebnisse sind in Tabelle 1, Spalte 5 und Tabelle 2 jeweils Spalte 3 jeder Gruppe (1 bis 4) dargestellt. Spalte 4 der Tabelle 1 zeigt, mit welchen Zytostatika der betreffende Patient behandelt worden war.

In Tabelle 2 sind für jeden Patienten die (vor Chemotherapiebeginn) ex-vivo mit den Zellen seiner Tumorgewebeprobe ermittelten Cisplatin-IC50-Werte dem in vivo und in situ Chemotherapieergebnis an seinem Tumor (mit Cisplatin- oder Carboplatin-Kombinationen) gegenüber gestellt. Diese Gegenüberstellung zeigt:
- Ex-vivo ermittelte IC50-Werte von unter 5 µM (Patientengruppe 1) korrelieren mit einer ausgeprägten Platinempfindlichkeit des Tumors in-vivo: Alle Tumoren in situ zeigen nach bzw. während der Chemotherapie eine partielle Remission (PR).
- Ex-vivo ermittelte IC50-Werte von größer oder gleich 5 µM und kleiner oder gleich 8 µM (Gruppe 2 der untersuchten Patienten) korrelieren mit einer mäßigen Platinempfindlichkeit des Tumors in-vivo: Die betreffenden Tumoren in situ zeigen nach bzw. während der Chemotherapie entweder eine partielle Remission (PR) oder zumindest Nullwachstum (NC, "no change")

Die Ergebnisse ex-vivo und in-vivo für die Tumorgewebeproben ID 278 und ID 279, die beide von demselben Patienten stammen (jedoch von zwei verschiedenen Tumoren dieses Patienten) verdeutlichen besonders die Unterscheidbarkeit zwischen Gruppe 1 und Gruppe 2 (der untersuchten Patienten), d.h. die Abgrenzbarkeit von ausgeprägter Platinempfindlichkeit (Gruppe 1) gegenüber mäßiger Platinempfindlichkeit (Gruppe 2): Der IC50-Wert der Tumorzellen der Tumorgewebeprobe ID 279 (eines Plattenepithelkarzinoms) betrug 3,73 µM und der betreffende Tumor in vivo (das Plattenepithelkarzinom) zeigte nach der Chemotherapie (mit Cisplatin kombiniert mit Gemzitabin) eine lang andauernde partielle Remission. Dagegen betrug der IC50-Wert der Tumorzellen der Tumorgewebeprobe ID 278 (eines Adenokarzinoms) 5,25 µM und der betreffende Tumor in vivo (das koexistierende Adenokarzinom) zeigte nach der derselben Chemotherapie (mit Cisplatin kombiniert mit Gemzitabin) lediglich Nullwachstum.

Die IC50-Werte der sieben Tumoren in Gruppe 2 (mäßige Platinempfindlichkeit) liegen eng um den Mittelwert von 6,73 µM. Im Stand der Technik bekannte pharmakokinetische Studien haben gezeigt, dass die mittlere vom Patienten maximal tolerierte Blutplasmakonzentration von Cisplatin bei fünftägiger Infusionsbehandlung 6,67 µM beträgt (Desoize et al., 1996).
Unter Berücksichtigung der natürlichen Schwankungen der Blutplasmakonzentration der Substanz ist bereits bei Tumoren mit ex vivo bestimmten IC50-Werten für Cisplatin über 6,7µM von einer beginnenden Unempfindlichkeit (Resistenz) des Tumors in vivo und in situ (im Patienten) gegenüber Cisplatin auszugehen.

Die in Gruppe 3 aufgelisteten Ergebnisse zeigen Patiententumoren, deren Zellen ex vivo/in vitro einen Cisplatin-IC50-Wert größer 8 µM aufwiesen.
Dennoch zeigten hier die betreffenden Tumoren ebenso wie ID 228 aus Patientengruppe 4 in vivo und in situ nach der Chemotherapie mit Cisplatin (in Kombination mit Gemzitabin oder Etoposid oder Pemetrexed und zum Teil weiteren Nicht-Platinzytostatika) einen Therapieerfolg in Form einer partiellen Remission oder eines Nullwachstum des Tumors. Dieser Befund lässt sich damit erklären, dass in diesen sechs Fällen die therapeutischen Effekte/Erfolge (PR, NC) auf dem Zusammenwirken der Nicht-Platinzytostatika Pemtrexed (ID 228, ID 297, ID 311, ID 314), Etoposid (ID 272) und Gemzitabin (ID 258) mit Cisplatin oder Carboplatin beruhten.
Prätherapeutische IC50-Bestimmungen für Cisplatin oder andere Platinanaloga sollten durch Assays ergänzt werden, die zeigen, ob in der Tumorprobe enthaltene platinresistente Bronchialkarzinomzellen mit Hilfe von Nicht-Platinzytostatika, insbesondere Pemetrexed, Etoposid und Gemzitabin, ex vivo platinsensibel gemacht werden können.

Gruppe (4) umfasst Sonderfälle:
Für ID 233 und ID 228 war die rechnerische Ermittlung der IC50-Werte anhand der log-linearen Plots nicht eindeutig möglich; sie lagen über 26 µM.
Bei ID 282 wurde die Chemotherapie ohne Einsatz von Platinderivaten/Platinanaloga allein mit Gemzitabin durchgeführt.

### (III) Erstellung der Datenbank und Informationsgehalt der Datenbank

Die in (I) und (II) ermittelten Daten bzw. Ergebnisse liefern folgende Informationen: Liegen die mit den epithelialen Tumorzellen einer Bronchialkarzinomgewebeprobe eines beliebigen aktuellen Patienten ex vivo im Labortest (vorzugsweise mit der Vorgehensweise nach I) bestimmten IC50-Werte für Cisplatin unter 5 µM, so ist zu erwarten, dass eine Cisplatin-Behandlung oder Behandlung mit anderen Platinderivaten/Platinanaloga bei dem betreffenden Patienten zur partiellen Remission seines Tumors (Bronchialkarzinoms) führt. Liegen diese IC50-Werte zwischen 5 µM und 6,7 µM, so weist das darauf hin, dass eine Chemotherapie des betreffenden Patienten mit Cisplatin oder anderen Platinderivaten/Platinanaloga mit hoher Wahrscheinlichkeit wenigstens zu einem Wachstumsstillstand des Tumors (Bronchialkarzinoms) führt.

IC50-Werte für Cisplatin über 6,7 µM bedeuten Resistenz des Tumors in vivo und in situ gegen alleinige Behandlung mit Cisplatin.

Mit diesen Daten lässt sich erfindungsgemäß eine Datenbank zur Abschätzung des Erfolgs einer geplanten Chemotherapie auf der Basis von Platinderivaten/Platinanaloga, insbesondere von Cisplatin, zwecks Bekämpfung eines Bronchialkarzinoms ("Therapieerfolgs-Prognose beim Bronchialkarzinom mit dem Zytostatikum Cisplatin") erstellen. Diese Datenbank liefert im gegenwärtigen Zustand die Informationen
1.) dass ein IC50-Wert für Cisplatin unter 5 µM (IC50 < 5 µM), der im Labor, d.h. ex vivo mit Tumorzellen (aus einer zuvor entnommenen Bronchialkarzinomgewebeprobe) des betreffenden Patienten ermittelt wurde, mit der Chemotherapieergebnisprognose "partielle Remission" bzw. "Tumorstadium Klasse II" korreliert, wenn diesem Patienten Cisplatin verabreicht wird; und
2.) dass ein IC50-Wert für Cisplatin zwischen 5 µM und 6,7 µM (5 µM ≤ IC50 < 6,7 µM), der im Labor, d.h. ex vivo und insbesondere in vitro, mit Tumorzellen (aus einer zuvor entnommenen Bronchialkarzinomgewebeprobe) des betreffenden Patienten ermittelt wurde, mit der Chemotherapieergebnisprognose zumindest "Nullwachstum" bzw. mindestens "Tumorstadium Klasse III" korreliert, wenn diesem Patienten Cisplatin verabreicht wird;
3.) dass ein IC50-Wert für Cisplatin über 6,7 µM Resistenz des entsprechenden Tumors beim Patienten gegen eine alleinige Cisplatin-Behandlung bedeutet. Therapieerfolge wie partielle Remission oder Nullwachstum des Tumors sind dann nur durch Kombination von Cisplatin mit anderen Zytostatika wie Gemzitabin, Etoposid und Pemetrexed erreichbar.

Weitere Datenerhebungen gemäß vorliegender Erfindungsbeschreibung können diese Hypothese bestätigen und/oder spezifizieren und/oder modifizieren.
Entsprechendes gilt für die zusätzliche Einbeziehung von Sensitizern wie Gemzitabin, Etoposid und Pemetrexed in die IC50-Bestimmung von Cisplatin oder anderen Platinanaloga.

Mit der Einspeisung weiterer Daten in diese Datenbank kann außerdem die Korrelation zwischen IC50-Wert und Chemotherapieerfolgsprognose weiter spezifiziert werden, beispielsweise in Unter- oder Zwischenklassen der Standard-Klassen I, II, III und IV.

Eine mögliche Darstellung der erfindungsgemäßen Datenbank "Therapieerfolgsprognose beim Bronchialkarzinom und Verabreichung des Zytostatikums Cisplatin" mit den gegenwärtigen verfügbaren Daten und Informationen zur Korrelation zwischen IC50-Wert (ex vivo/in vitro) und Chemotherapieerfolg (in vivo/in situ) ist in Form der Tabelle 3 gegeben.

Platinverbindungen bilden die Grundlage der Chemotherapie nicht nur bei Bronchialkarzinomen, sondern auch bei vielen anderen epithelialen Tumoren. Die Höhe der vom Patienten vertragenen Platindosis richtet sich dabei nach der Toxizität der Substanz für zentrale Organismusfunktionen, etwa die Bildung weißer Blutzellen im Knochenmark. Die individuell vorhandene Tumorart ist dabei unerheblich.

Nach alledem ist zu erwarten, dass die in der Praxis beim Bronchialkarzinom ermittelten quantitativen Beziehungen zwischen der Cisplatinempfindlichkeit der Tumorzellen ex vivo (messbar im Labortest) und der Empfindlichkeit des Tumors in vivo gegenüber einer Cisplatin-basierten Chemotherapie des Patienten ebenso für andere mit Cisplatinverbindungen behandelbare Tumoren beim Menschen (und eventuell auch Tier) gelten.

Eine nach (I) und (II) erstellte erfindungsgemäße Datenbank gibt dem zuständigen Therapeuten somit ein Mittel an die Hand, mit dem er auf relative einfach Weise und in ausreichend kurzer Zeit einen aussagekräftigen Hinweis darauf erhält, ob eine geplante Platin-basierte, insbesondere Cisplatin-basierte Therapie bei dem betreffenden individuellen Bronchialkarzimom des betreffenden individuellen Patienten voraussichtlich erfolgreich sein wird und in welchem Ausmaß (Tendenz "nur" Stillstand des Tumorwachstums oder Tendenz "Remission/Rückbildung" des Tumors).

Analog erstellte Datenbanken für andere Tumorentitäten stellen entsprechende Hilfsmittel für die jeweiligen anderen Tumorentitäten dar.

Dabei ist vorstellbar, dass sich herausstellt, dass jedenfalls solche Tumorentitäten, die auch bisher große Ähnlichkeiten mit Bronchialkarzinomen gezeigt haben, z.B. das Ovarialkarzinom, praktisch gleiche Korrelationen zwischen den ex vivo/in vitro ermittelten IC50-Werten und dem Tumorstadium in vivo und in situ nach verabreichter Chemotherapie zeigen, insbesondere im Fall einer platin-basierten Chemotherapie.

### Zitierte Literatur:

WO 2009/124997 A1 "Method and kit for the ex vivo evaluation of the response of a tumor to conditions to be tested"
DE 102012003700 B3 "Verfahren zur Trennung von Verbänden maligner Zellen und Verbänden aus Stromazellen einer Malignomgewebeprobe"
Dollner R, Granzow C, Helmke BM, Ruess A, Schad A, Dietz A: The impact of stromal cell contamination on chemosensitivity testing of head and neck carcinoma. Anticancer Res. 24: 325-31 (2004)
Granzow C, Kopun M, Heuser M, Herth F, Becker HD: Chemoresistance of human lung tumor stromal cells. Amer. Assn. Cancer Res. 95th Annual Meeting Proc. Suppl., abstract LB-82 (2004)
Heuser M, Kopun M, Rittgen W, Granzow C: Cytotoxicity determination without photochemical artefacts. Cancer Lett. 223: 57-66 (2005)
Desoize B, Berthiot G, Manot L, Coninx P, Dumont P: Evaluation of a prediction model of cisplatin dose based on total platinum plasma concentration. Eur J Cancer 32A: 1734-8 (1996)

**Tabelle 1: Pathohistologische Klassifizierung und Stadium des Tumors, verabreichte Chemotherapie und Therapieerfolg bei 18 Bronchialkarzinom-Tumorproben von 17 Patienten**

| | | **Pathohistologie/** | | |
|---|---|---|---|---|
| **Lfd. Nr.** | **ID** | **Tumorstadium "St."** | **Verwendete Cytostatika** | **Therapieerfolg** |
| 1 | **220** | SCLC / St. IV | CarboPt, Etoposid | PR |
| 2 | **221** | NSCLC / St. IV | CisPt, Gemzitabin | PR |
| 3 | **228** | NSCLC / St. IV | CarboPt, Pemetrexed | PR |
| 4 | **233** | NSCLC / St. IV | CisPt, CarboPt, Etoposid, Docetaxel | Progress |
| 5 | **245** | NSCLC / St. IV | CarboPt, Gemzitabin | PR |
| 6 | **255** | SCLC / St. IV | CarboPt, Etoposid | PR |
| 7 | **258** | NSCLC / St. IV | CisPt, Gemzitabin | PR |
| 8 | **260** | NSCLC/ St. Illb | CisPt, CarboPt, Gemzitabin | PR |
| 9 | **272** | SCLC /St. IV | CisPt, Etoposid | NC |
| 10 | **275** | NSCLC / St. IV | CarboPt, Gemzitabin | NC |
| 11 | **277** | NSCLC / St. IIIa | CarboPt, Vinorelbin | NC |
| 12 | **278** | NSCLC / St. IV | CisPt, Gemzitabin | NC |
| 13 | **279** | NSCLC / St. IV | CisPt, Gemzitabin | PR |
| 14 | **282** | NSCLC / St. IV | Gemzitabin | Progress |
| 15 | **294** | NSCLC / St. IV | CarboPt, Pemetrexed | NC |
| 16 | **297** | NSCLC / St. IV | CisPt, Pemetrexed, LY2603618 | NC |
| 17 | **311** | NSCLC / Kl. IIIb | CisPt, Pemetrexed | NC |
| 18 | **314** | NSCLC / Kl. IV | CisPt, CarboPt, Pemetrexed | PR |

**Tabelle 2: Korrelation der prä-therapeutisch ermittelten Cisplatin-IC50-Werte der Tumorzellen ex vivo mit den Ergebnissen der platinbasierten Chemotherapie beim Patienten in vivo**

| **Cisplatinresponse ex vivo** | | | | | | | | | | | |
|---|---|---|---|---|---|---|---|---|---|---|---|
| **Gruppe 1 IC50 < 5 µM** | | | **Gruppe 2 IC50 ≥ 5 < 8 µM** | | | **Gruppe 3 IC50 ≥ 8 bis 25,6 µM** | | | **Gruppe 4 Sonstige** | | |
| **ID** | **IC50** | **Chemo-response** | **ID** | **IC50** | **Chemo-response** | **ID** | **IC50** | **Chemo-response** | **ID** | **IC50** | **Chemo-response** |
| **220** | 2,60 | PR | **278** | 5,25 | NC | **258** | 16,72 | PR | **282** | 14,8 | Progress |
| **279** | 3,73 | PR | **275** | 5,38 | NC | **314** | 20,51 | PR | **228** | > 26 | PR |
| **255** | 4,58 | PR | **260** | 5,61 | PR | **272** | 21,01 | NC | **233** | > 26 | Progress |
| | | | **277** | 7,58 | NC | **311** | 22.09 | NC | | | |
| | | | **294** | 7,70 | NC | **297** | 25,60 | NC | | | |
| | | | **245** | 7,74 | PR | | | | | | |
| | | | **221** | 7,77 | PR | | | | | | |
| | | | | | | | | | | | |
| Mittelwert 3,64 | | | Mittelwert 6,72 | | | Mittelwert 21,12 | | | | | |
| Standardabweichung 0,99 | | | Standardabweichung 1,23 | | | Standardabweichung 3,19 | | | | | |
| p-Wert Gruppe **1 zu 2** =0,0095 | | | p-Wert Gruppe **2 zu 3** =0,0002 | | | | | | | | |

**Tabelle 3: Datenbank " Therapieerfolgsprognose beim Bronchialkarzinom und Verabreichung des Zytostatikums Cisplatin "**

| prä-therapeutisch ex vivo / in vitro ermittelte **IC50-Werte** der malignen Tumorzellen für **Cisplatin** | Prognose des in vivo-Effekts der Cisplatin-basierten Chemotherapie beim Patienten |
|---|---|
| 0 µM < IC50 < ...?... µM | Klasse I (bisher nicht beobachtet) |
| ...?... µM < IC50 < 5 µM | Klasse II |
| 5 µM ≤ IC50 ≤ 6,7 µM | Klasse II-III (Kombinationstherapie erforderlich |
| 6,7 µM < IC50 <...?... | Klasse II-IV (Kombinationstherapie erforderlich |

| | |
|---|---|
| Klasse I = vollständige Remission des Tumors (VR), Klasse II = partielle Remission des Tumors (PR) Klasse III = Nullwachstum des Tumors = "no change" (NC) Klasse IV = fortschreitendes Wachstum des Tumors = Progress | |

## Patentansprüche

1. Verfahren zur Erstellung einer Datenbank für die Vorabschätzung der Wirksamkeit eines zytostatischen Wirkstoffs, der ein Platinderivat/Platinanalogon ist, in der chemotherapeutischen Behandlung eines Tumors, insbesondere eines Malignoms, eines bestimmten Patienten anhand der ex vivo Ermittlung des IC50 Wertes des Wirkstoffs für die Tumorzellen einer zuvor entnommenen Tumorgewebeprobe desselben Patienten, **gekennzeichnet durch** folgende Maßnahmen:
- aus der Tumorgewebeprobe wird durch enzymatischen Verdau mit Kollagenase bei 36-37°C für 4-5 Stunden, Zentrifugieren und Resuspendieren ein Tumordigest hergestellt, das Zellverbände aus malignen Zellen einerseits und Stromazellen, d. h. Endothelzellen und Fibroblasten, andererseits enthält,
- die Zellverbände werden ex vivo mit dem betreffenden Wirkstoff inkubiert durch Zugabe von Aliquots dieses Tumordigests zu vorab doppelt konzentriert angesetzten gleichgroßen Aliquots von Wirkstoff in Kulturmedium;
- der IC50-Wert dieses Wirkstoffs bei den malignen Zellen wird ermittelt als diejenige Konzentration des Wirkstoffs, bei der im Vergleich zu Negativ-Kontrolle (0 µM Wirkstoff) eine halbmaximale Inhibition der Koloniebildung beobachtbar ist;
- dem ex vivo ermittelten IC50-Wert wird die Dokumentation und/oder Klassifizierung desjenigen Therapieergebnisses zugeordnet, das von demselben Patienten nach Therapie mit demselben Wirkstoff gemäß geltenden evidenz-basierten Therapieregeln stammt und das dokumentiert und klassifiziert worden ist,
- die verwendeten Wirkstoffkonzentrationen werden mittels parallel untersuchter etablierter in vitro Zellstämme der gleichen Tumorentität verifiziert.

2. Verfahren nach Anspruch 1, **dadurch gekennzeichnet, dass** die Klassifizierung des dokumentierten Therapieergebnisses in Form der Klassen I bis IV erfolgt, wobei gilt: Klassen I = vollständige Remission des Tumors (VR), II = partielle Remission des Tumors (PR), III = Nullwachstum des Tumors = "no change" (NC) und IV = fortschreitendes Wachstum des Tumors = Progress.

3. Verfahren nach einem der Ansprüche 1 oder 2, **dadurch gekennzeichnet, dass** die zur Ermittlung des IC50-Werts eingesetzten Zellen maligne Zellen sind, die zuvor aus der entnommenen Tumorgewebeprobe ex vivo separiert wurden.

4. Verwendung einer gemäß einem der Ansprüche 1 bis 3 erstellten Datenbank in einem Verfahren zur Voreinschätzung der Wirksamkeit eines zytostatischen Wirkstoffs oder einer Wirkstoff-Kombination, wobei der oder einer der Wirkstoffe ein Platinderivat/Platinanalogon ist, zur Bekämpfung des Tumors eines Patienten anhand der ex vivo Ermittlung des IC50 Wertes des Wirkstoffs oder der Wirkstoff-Kombination für die Tumorzellen einer zuvor entnommenen Tumorgewebeprobe des betreffenden Patienten,
wobei der bei den gemäß Anspruch 1 gewonnenen Tumorzellverbänden der zuvor entnommenen Tumorgewebeprobe desselben Patienten ex vivo nach Inkubation mit dem betreffenden Wirkstoff oder der Wirkstoff-Kombination durch Zugabe von Aliquots des Tumordigests zu vorab doppelt konzentriert angesetzten gleichgroßen Aliquots von Wirkstoff oder Wirkstoffkombination in Kulturmedium ermittelte IC50-Wert der malignen Zellen mit den in der Datenbank für diesen Wirkstoff oder diese Wirkstoff-Kombination enthaltenen IC50-Referenzwerten verglichen wird, wobei der IC50 Wert diejenige Konzentration des Wirkstoffs oder der Wirkstoffskombination ist, bei der im Vergleich zur Negativ-Kontrolle (0µM Wirkstoff oder Wirkstoffkombination) eine halbmaximale Inhibition der Koloniebildung beobachtbar ist, und wobei Übereinstimmung mit einem IC50-Referenzwert ein Indiz dafür ist, dass das in der Datenbank diesem IC50-Referenzwert zugeordnete Therapieergebnis auch im Fall einer Behandlung des aktuellen Patienten mit diesem Wirkstoff oder dieser Wirkstoff-Kombination eintritt.

5. Verfahren zur Vorabschätzung der Wirksamkeit eines zytostatischen Wirkstoffs, der ein Platinderivat/Platinanalogon ist, in der chemotherapeutischen Behandlung eines Tumors, insbesondere eines Malignoms, eines bestimmten aktuellen Patienten, bei dem
- die aus einer zuvor entnommenen Tumorgewebeprobe des Patienten durch enzymatischen Verdau mit Kollagenase bei 36-37°C für 4-5 Stunden, Zentrifugieren und Resuspendieren gewonnenen Zellverbände aus malignen Zellen einerseits und Stromazellen, d.h. Endothelzellen und Fibroblasten, andererseits ex vivo mit dem betreffenden Wirkstoff inkubiert werden durch Zugabe von Aliquots dieses Tumordigests zu vorab doppelt konzentriert angesetzten gleichgroßen Aliquots von Wirkstoff in Kulturmedium, und der IC50-Wert dieses Wirkstoffs bei den malignen Zellen ex vivo als diejenige Konzentration des Wirkstoffs ermittelt wird, bei der im Vergleich zu Negativ-Kontrolle (0 µM Wirkstoff) eine halbmaximale Inhibition der Koloniebildung beobachtbar ist,
- der ermittelte IC50-Wert verglichen wird mit parallel ermittelten IC50-Referenzwerten des gleichen Wirkstoffs bei in vitro Zellstämmen der gleichen Tumorentität, wobei jedem IC-50-Referenzwert ein Therapieergebnis zugeordnet ist, das von demjenigen Patient stammt, mit dessen Tumorzellen der IC50-Refernzwert gewonnen wurde, nach Behandlung mit diesem Wirkstoff gemäß geltenden evidenz-basierten Therapieregeln, und das dokumentiert und klassifiziert worden ist, beispielsweise in Form einer Klassifizierung in eine der Klassen I = vollständige Remission des Tumors (VR), II = partielle Remission des Tumors (PR), III = Nullwachstum des Tumors = "no change" (NC) und IV = fortschreitendes Wachstum des Tumors = Progress,
- und Übereinstimmung zwischen dem IC50-Wert bei den Tumorzellen des aktuellen Patienten und einem IC50-Referenzwert als Indiz dafür gilt, dass das zu diesem Referenzwert gehörende Therapieergebnis auch im Fall einer Behandlung des aktuellen Patienten eintritt.

## Claims

1. Method for creating a database for the pre-assessment of the efficacy of a cytostatic active substance, which is a platinum derivative/platinum analogoue, in the chemotherapeutic treatment of a tumor, especially a malignant tumor, of a particular patient on the basis of ex vivo determination of the IC50 value of the active substance for the tumor cells of a tumor tissue sample previously taken from the same patient, comprising the following measures:
- from the tumor tissue sample a tumor digest is prepared by carrying out an enzymatic digestion with collagenase at 30-37°C for 4-5 hours, centrifugation and resuspension, wherein said tumor digest contains cell clusters of malignant cells on the one hand and stroma cells, i.e. endothelial cells and fibroblasts on the other,
- the cell clusters are incubated ex vivo with the active substance concerned by addition of aliquots of this tumor digests to previously prepared twice concentrated equal aliquots of active substance in culture medium,
- the IC50 value of this active substance in the malignant cells is determined as the concentration of the active substance at which a half-maximal inhibition of colony formation is observably as compared to negative controls (0 µM active substance),
- the ex vivo determined IC50-value is assigned to the documentation and/or classification of the therapy outcome which has been obtained with the same patient after treatment according to applicable evidence based therapy rules with the same active substance, and which has been documented and classified.
- the applied concentrations of the active substance are verified by means of established in vitro cell strains of the same tumour entity which are examined in parallel.

2. Method according to claim 1, **characterized in that** the classification of the documented therapy outcome is done in the form of classes I to IV, with classes I = complete (vollständige) remission of the tumor (VR), II = partial remission of the tumor (PR), III = zero growth of the tumor - "no change" (NC), and 1V = progressive tumor growth = Progress.

3. Method according to one of claims 1 or 2, **characterized in that** the cells used to determine the IC50-value are malignant cells previously separated ex vivo from the tumor tissue sample taken.

4. Use of a database created according to any one of claims 1 to 3 in a process for pre-assessment of the efficacy of a cytostatic active substance or a combination of active substances, wherein the or one of the active substance(s) is a platinum derivative/platinum analogous, to combat the tumor of a patient based on the ex vivo determination of the IC50-value of the active substance or the combination of active substances for the tumor cells of a tumor tissue sample previously taken from the patient concerned,
wherein the IC50-value of the malignant cells, determined with the tumor cell clusters obtained from the tumor tissue sample previously taken from the same patient according to claim 1, ex vivo after incubation with the active substance or combination of active substances concerned by addition of aliquots of the tumor digests to previously prepared twice concentrated equal aliquots of the active substance or combination of active substances in culture medium, is compared with the reference IC50-values contained in the database for this active substance or combination of active substances,
- wherein the IC50 value is that concentration of the active substance or combination of active substances, at which a half-maximal inhibition of colony formation is observably as compared to the negative control (0 µM active substance or combination of active substances),
and wherein any match with a reference IC50-value is an indication that the therapy outcome assigned in the database to this reference IC50-value also will occur in the case of treatment of the current patient with this active substance or combination of active substances.

5. Method for the pre-assessment of the efficacy of a cytostatic active substance which is a platinum derivative/platinum analogoue in the chemotherapeutic treatment of a tumor, particularly a malignant tumor, of a certain current patient, wherein
- the cell clusters of malignant cells on the one hand and stroma cells, i.e. endothelial cells and fibroblasts on the other, which were obtained from a previously taken tumor tissue sample of the patient by enzymatic digestion with collagenase at 36-37°C for 4-5 hours, centrifugation and resuspension, are ex vivo incubated with the active substance concerned by addition of aliquots of this tumor digests to previously prepared twice concentrated equal aliquots of active substance in culture medium, and the IC50-value of this active substance with the malignant cells is determined ex vivo as the concentration of the active substance at which a half-maximal inhibition of colony formation is observably as compared to negative controls (0 µM active substance),
- the determined IC50-value is compared with reference IC50-values of the same active substance determined in parallel with in vitro cell strains of the same tumour entity, wherein each reference IC50-value is assigned to a therapy outcome, which originates from the patient with whose tumor cells the reference IC50-value was obtained after treatment with this active substance according to applicable evidence-based therapy rules, and which has been documented and classified, for example in form of a classification in one of the classes I - complete (vollständige) remission of the tumor (VR), 11 - partial remission of the tumor (PR), III = zero growth of the tumor = "no change" (NC), and IV = progressive tumor growth = Progress.
- and any match between a reference IC50-value and the IC50-value with the tumor cells of the current patient is an indication that the therapy outcome related to this reference IC50-value also will occur in the case of treatment of the current patient.

## Revendications

1. Procédé de préparation d'une base de données pour la pré-évaluation de l'efficacité d'une substance active cytostatique qui est un dérivé de platine/analogue de platine, pour le traitement chimiothérapeutique d'une tumeur, en particulier un malignome, d'un patient spécifique sur la base de la détermination ex vivo de la valeur CI50 de la substance active pour les cellules tumorales d'un échantillon de tissu tumoral prélevé préalablement du même patient, **caractérisé par** les dispositions suivantes :
- une digestion tumorale est produite à partir de l'échantillon de tissu tumoral par digestion enzymatique avec de la collagénase à 36-37 °C pendant 4-5 heures, centrifugation et resuspension, laquelle digestion tumorale contient des amas cellulaires de cellules malignes d'une part et de cellules stromales, c'est-à-dire de cellules endothéliales et de fibroblastes, d'autre part,
- les amas cellulaires sont incubés ex vivo avec la substance active concernée en ajoutant des aliquotes de cette digestion tumorale à des aliquotes de même taille précédemment doublement concentrées de substance active dans le milieu de culture ;
- la valeur CI50 de cette substance active pour les cellules malignes est déterminée comme étant la concentration de la substance active à laquelle une inhibition médiane de la formation de colonies peut être observée par rapport au témoin négatif (0 µM de substance active) ;
- la valeur CI50 déterminée ex vivo est affectée à la documentation et/ou à la classification du résultat de la thérapie qui a été obtenue avec le même patient après une thérapie avec la même substance active selon des règles thérapeutiques fondées sur des données probantes valables et qui a été documentée et classifiée,
- les concentrations de substance active utilisées sont vérifiées au moyen de souches cellulaires in vitro établies de la même entité tumorale et examinées en parallèle.

2. Procédé selon la revendication 1, **caractérisé en ce que** la classification du résultat thérapeutique documenté est réalisée sous la forme de classes I à IV, où : classes I = rémission complète de la tumeur (VR), II = rémission partielle de la tumeur (PR), III = croissance nulle de la tumeur ="pas de changement" (NC) et IV = croissance progressive de la tumeur = progression.

3. Procédé selon l'une des revendications 1 ou 2, **caractérisé en ce que** les cellules utilisées pour déterminer la valeur CI50 sont des cellules malignes qui ont été préalablement séparées ex vivo de l'échantillon de tissu tumoral prélevé.

4. Utilisation d'une base de données préparée conformément aux revendications 1 à 3 dans un procédé de pré-évaluation de l'efficacité d'une substance active cytostatique ou d'une combinaison de substances actives, la substance active ou l'une des substances actives étant un dérivé du platine/analogue du platine pour le traitement de la tumeur d'un patient sur la base de la détermination ex vivo de la valeur CI50 de la substance active ou de la combinaison de substances actives pour les cellules tumorales d'un échantillon de tissu tumoral préalablement prélevé du patient concerné,
la valeur CI50 des cellules malignes pour les amas de cellules tumorales obtenus selon la revendication 1 de l'échantillon de tissu tumoral du même patient prélevé préalablement est comparée, ex vivo après incubation avec la substance active concernée ou la combinaison de substances actives par addition d'aliquotes de la digestion tumorale à des aliquotes de taille égale de substance active ou combinaison de substances actives précédemment doublement concentrées en milieu de culture , aux valeurs de référence CI50 contenues dans la base de données pour cette substance active ou cette combinaison de substances actives,
la valeur CI50 est la concentration de la substance active ou de la combinaison de substances actives à laquelle une inhibition médiane de la formation de colonies peut être observée en comparaison avec le témoin négatif (0 µM de substance active ou de la combinaison de substances actives), et
la concordance avec une valeur de référence CI50 étant un indice que le résultat thérapeutique associé à cette valeur de référence CI50 dans la base de données se produira également dans le cas d'un traitement du patient actuel avec cette substance active ou cette combinaison de substances actives.

5. Méthode pour pré-évaluer l'efficacité d'une substance active cytostatique qui est un dérivé du platine/analogue du platine dans le traitement chimiothérapeutique d'une tumeur, en particulier d'un malignome, d'un patient actuel particulier, méthode dans laquelle :
- d'une part les amas cellulaires de cellules malignes, obtenus à partir d'un échantillon de tissu tumoral prélevé préalablement sur le patient par digestion enzymatique avec de la collagénase à 36-37 °C pendant 4-5 heures, centrifugation et resuspension et, et d'autre part des cellules stromales, à savoir des cellules endothéliales et des fibroblastes, sont mises à incuber ex vivo avec la substance active concernée en ajoutant des aliquotes de cette digestion tumorale à des aliquotes de même taille préalablement doublement concentrées de la substance active dans le milieu de culture, et la valeur CI50 de cette substance active dans les cellules malignes est déterminée ex vivo comme étant la concentration de la substance active à laquelle une inhibition médiane de la formation de colonies peut être observée en comparaison avec le contrôle négatif (0 µM de substance active),
- la valeur CI50 déterminée est comparée aux valeurs de référence CI50 de la même substance active déterminées parallèlement pour des souches cellulaires in vitro de la même entité tumorale, chaque valeur de référence CI-50 étant associée à un résultat thérapeutique qui a été obtenu avec ledit patient avec les cellules tumorales duquel la valeur de référence CI50 a été obtenue, après traitement avec cette substance active selon des règles thérapeutiques fondées sur des données probantes valables, et qui a été documenté et classé, par exemple sous la forme d'une classification dans l'une des classes I = rémission complète de la tumeur (VR), II = rémission partielle de la tumeur (PR), III = croissance nulle de la tumeur = "pas de changement" (NC) et IV = croissance progressive de la tumeur = progression, et
- une concordance entre la valeur CI50 dans les cellules tumorales du patient actuel et une valeur de référence CI50 étant un indice que le résultat thérapeutique appartenant à cette valeur de référence se produira également dans le cas d'un traitement du patient actuel.
